# EUROPEAN PATENT SPECIFICATION

(11) **EP 0 306 190 B1**
(45) Date of publication and mention of the grant of the patent: **08.04.1998**
(21) Application number: 88307750.5
(22) Date of filing: 22.08.1988
(51) Int. Cl.: C07H 7/027, C07H 13/08, C07H 19/073, C07H 19/04, C07D 307/32

(54) **Process for preparing intermediates useful in the preparation of 2',2'-difluoronucleosides**
Verfahren zur Herstellung von Zwischenverbindungen verwendbar in der Herstellung von 2',2'-Difluoronucleosiden
Procédé pour la préparation de composés intermédiares utilisable pour la préparation de 2',2'-Difluoronucléosides

(30) Priority: 28.08.1987 US 90725
(43) Date of publication of application: 08.03.1989
(60) Divisional application: 94202041.3
(73) Proprietor: ELI LILLY AND COMPANY, Indianapolis, Indiana 46285 (US)
(72) Inventor: Chou, Ta-Sen, Indianapolis, IN 46217 (US); Heath, Perry Clark, Indianapolis, IN 46227 (US); Patterson, Lawrence Edward, Indianapolis, IN 46226 (US)
(74) Representative: Tapping, Kenneth George

(56) References cited:
- EP-A- 0 122 707
- EP-A- 0 184 365
- EP-A- 0 211 354

## Description

This invention provides a novel process for preparing an enatiomeric mixture of erythro and threo lactones which is useful in the preparation of 2',2'-difluoronucleosides.

United States Patent No. 4,526,988 teaches that 2'-deoxy-2',2'-difluoronucleosides are useful antiviral agents. European Patent Application 184,365 teaches the use of the same compounds as oncolytic agents. The synthetic process disclosed in the publications produces intermediates containing up to two centers of chirality. One such intermediate having a chiral center is a protected lactone consisting of erythro and threo enantiomers of the formulae 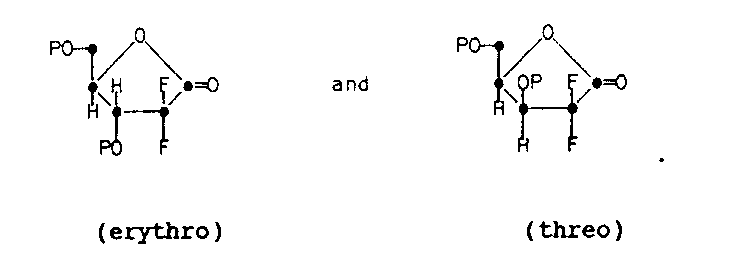 wherein P is a protecting group. The publications teach the erythro enantiomer is preferred since it provides a carbohydrate which has the stereochemistry of naturally occurring ribose. A carbohydrate which has the stereochemistry of naturally occurring ribose is preferred since it provides final product nucleosides which exhibit superior biological activity.

United States Patent No. 4,526,988 teaches the preparation of the above described erythro enantiomer by first forming an alkyl 2,2-difluoro-3-hydroxy-3-(2,2-dialkyldioxolan-4-yl)propionate, consisting of 3-R- and 3-S- hydroxy enantiomers, of the formulae 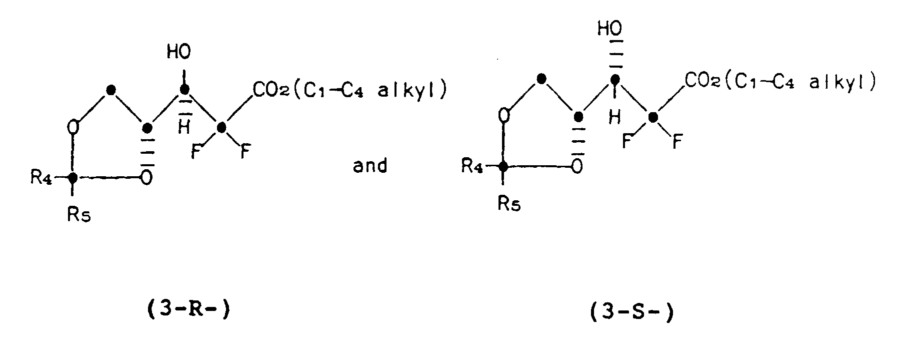 wherein R⁴ and R⁵ are independently C₁-C₃ alkyl, in a ratio of about 3 parts 3-R- enantiomer to about 1 part 3-S- enantiomer. The publication discloses that the 3-R- hydroxy enantiomer has the proper stereochemistry to provide the desired erythro enantiomer and that the 3-R- and 3-S- enantiomers can be separated by expensive, laborious column chromatography procedures.

The patent teaches that once the 3-R- hydroxy enantiomer is isolated it is next hydrolyzed under very mild conditions to form an unprotected lactone; namely, 2-deoxy-2,2-difluoro-D-erythro-pentofuranos-1-ulose, which has the formula 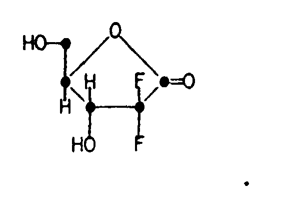 The publication teaches that mild conditions useful for forming the above compound include the use of hydrolysis reagents such as mildly acidic ion exchange resins or relatively strong acids, such as aqueous acetic acid or chloroacetic acid, which have a pKa between about 2.8 to about 5.0. Both types of hydrolysis reagents can cause problems in the hydrolysis reaction. For example, the use of ion exchange resin requires such large quantities of water that, especially in larger scale reactions, the lactone often reverts back to its open chain precursor because of its sensitivity to water. The relatively strong acids, on the other hand, are less preferred hydrolysis reagents for converting the 3-R- hydroxy enantiomer to the unprotected lactone since they produce large amounts of undesirable reaction products, including unreacted starting material.

Finally, once the unprotected lactone has been formed it is converted to the protected erythro lactone described above by adding an hydroxy protecting group to the lactone's hydroxy groups.

A second chiral center is produced at the anomeric carbon atom when the keto portion of the lactone is converted to an alcohol. More specifically, the two anomers for the desired erythro configuration are identified as α and β anomers of the fomulae 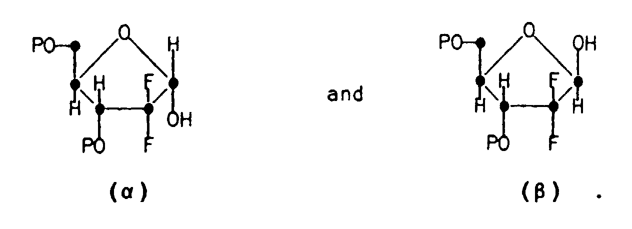 The unprotected hydroxy group at the 1-position is ultimately replaced by a heterocyclic base, such as cytosine, to provide protected precursors of the biologically active 2'-deoxy-2',2'-difluoronucleosides. The β anomer precursor is preferred since it provides 2'-deoxy-2',2'-difluoronucleosides which possess superior biological activity.

United States Patent No. 4,526,988 specifically illustrates the use of t-butyldimethylsilyl as a protecting group. When this protecting group is used in the synthesis of 2'-deoxy-2',2'-difluoronucleosides the product is composed of about a 4:1 α/β anomeric ratio. This product must be purified by expensive, laborious column chromatography procedures to isolate the desired β anomer.

The present invention provides a convenient process for obtaining an enatiomeric mixture of erythro and threo lactones which is useful in the preparation of 2'-deoxy-2',2'-difluoronucleosides.

According to the present invention there is provided a process for preparing an enantiomeric mixture of erythro and threo lactones of the formula 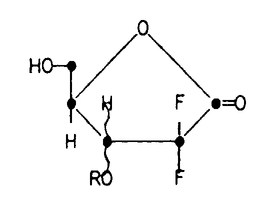 wherein R is H or 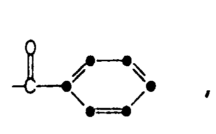 which comprises hydrolyzing a mixture of 3-R- and 3-S- enantiomers of an alkyl 2,2-difluoro-3-hydroxy-3-(2,2-dialkyldioxolan-4-yl)propionate, or a protected derivative thereof, of the formula 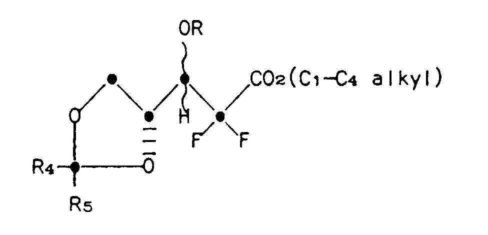 wherein R is as defined above and R⁴ and R⁵ are independently C₁-C₃ alkyl, by using a strong acid as a hydrolytic reagent, followed by azeotropic distillation of water. The present process prepares a crystalline lactone which is stable, therefore minimizing reversion back to the open chain precursor, as well as minimizing the formation of undesirable reaction products.

United States Patent No. 4,526,988 discloses alkyl 2,2-difluoro-3-hydroxy-3-(2,2-dialkyldioxolan-4-yl)propionates of the formula 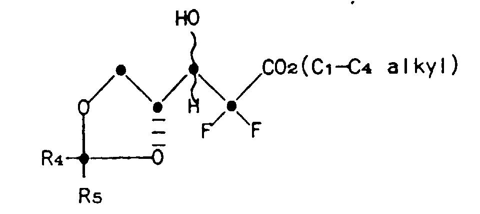 wherein R⁴ and R⁵ are independently C₁-C₃ alkyl. The compounds consist of 3-R- and 3-S- hydroxy enantiomers in a ratio of about 3 parts 3-R- enantiomer to 1 part 3-S- enantiomer. The present invention provides a process for converting the above compounds, or protected derivatives thereof, of the formula 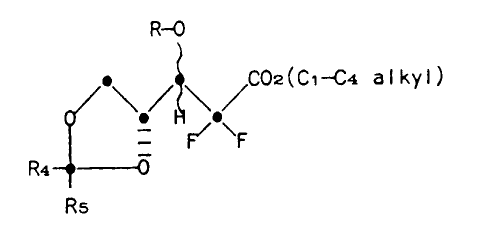 wherein R is H or 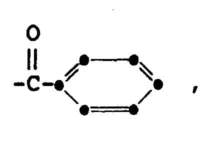 to a lactone of the formula

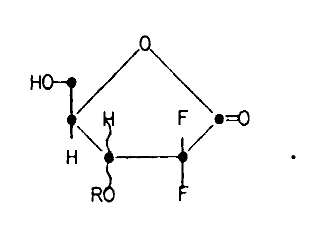

The protected derivative starting material noted above can be prepared by reacting the unprotected alkyl 2,2-difluoro-3-hydroxy-3-(2,2-dialkyldioxolan-4-yl)propionate with benzoyl bromide, chloride, cyanide, or azide. The reaction is conveniently carried out at temperatures in the range of from about -10°C to about 50°C in an inert solvent to which an acid scavenger, such as a tertiary amine, has been added. The reaction may also be carried out in a basic solvent such as pyridine, quinoline, isoquinoline, or lutidine, or in a tertiary amine solvent such as triethylamine, tributylamine, methylpiperidine, or the like. Additionally, a catalyst such as 4-dimethylaminopyridine or 4-pyrrolidinopyridine may be used in the reaction, if desired.

The 3-hydroxy compounds, or their benzoyl protected derivatives, are converted to the lactone in the following manner. First, the isoalkylidene protecting group is selectively removed to form an alkyl 2,2-difluoro-3,4,5-trihydroxypentanoate or alkyl 2,2-difluoro-3-(benzoyloxy)-4,5-dihydroxypentanoate compound of the formula

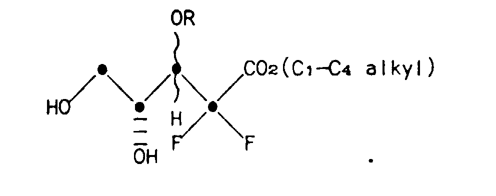

The selective removal of the isoalkylidene protecting group is achieved by using a strong acid as a hydrolytic reagent. The term "strong acids", as defined herein, are acids which have a pKa at room temperature (22°C) of about -10.0 to about 2.0. Examples of strong acids include inorganic acids such as 1 to 8 normal hydrochloric acid, 1 to 8 normal sulfuric acid, and the like, and organic acids such as p-toluenesulfonic acid, trifluoroacetic acid, and the like. Preferred strong acids are those acids which have a pKa of about -7.0 to about 0.0. Particularly preferred strong acids are 6N sulfuric acid, trifluoroacetic acid and p-toluenesulfonic acid. The strong acid is generally employed in catalytic quantities, although greater than catalytic quantities can be employed if desired. Typically the acid is employed in an amount sufficient to provide about 0.05 to about 0.5 molar equivalents of acid relative to the alkyl 2,2-difluoro-3-hydroxy-3-(2,2-dialkyldioxolan-4-yl)propionate, or protected derivative, starting material.

The propionate starting material and the strong acid are dissolved in a suitable solvent and the water content of the solution is adjusted to provide from about 1 to about 5 molar equivalents of water relative to the propionate starting material. Suitable solvents include polar solvents such as the alcohols, for example methanol, ethanol, isopropanol, and the like; acetonitrile; and related polar solvents. The water content of the solution can be adjusted to provide between about 1 to about 5 equivalents of water in several ways; by adding additional water to the water already present in the organic or inorganic strong acid, by choosing an inorganic acid which has the proper normality to provide the desired quantity of water, or by choosing a solvent, such as 95% ethanol, which contains a small amount of water. In general, about 1 to 2 molar equivalents of water relative to the propionate starting material are preferred since the lower water content is easily removed when cyclizing to the lactone.

After the propionate starting material, the strong acid, the solvent and water have been mixed, the solution is heated in order to begin selective removal of the isoalkylidene protecting group. The solution is preferably heated to the reflux temperature of the reaction mixture. The isoalkylidene protecting group is substantially removed after about 2 hours to about 8 hours when the reaction is conducted at the preferred temperature.

Once the isoalkylidene protecting group has been substantially removed the resulting pentanoate is cyclized to the desired lactone. The pentanoate is cyclized by distilling a water/alcohol, a water/acetonitrile, or a water/acetonitrile/aromatic solvent azeotropic mixture in order to remove water from the reaction solution. When an alcohol is used as the solvent the water/alcohol distillation preferably should continue until substantially all of the water and alcohol have been removed. However, when acetonitrile is used as the solvent fresh acetonitrile and/or aromatic solvent is added in order to ensure that sufficient solvent is present in order to drive out the water and any non-solvent volatile components, yet still maintain a homogeneous liquid solution. Preferably, an aromatic solvent, such as toluene, is used in place of fresh acetonitrile when removing water from an acetonitrile solvent solution since less solvent is then required to azeotropically dry the solution. Once the water has been substantially removed from the reaction mixture the pentanoate cyclizes to the lactone in high yield. This cyclization reaction can be monitored by high performance liquid chromatography assay techniques in order to determine when the reaction is substantially complete. The lactone produced consists of erythro and threo enantiomers in approximately the same enantiomeric proportions as present in the propionate starting material.

The following Examples illustrate specific aspects of the present invention. The Examples are not intended to limit the scope of the invention in any respect and should not be so construed.

### Example 1

### Preparation of an enantiomeric mixture of D-erythro- and D-threo-2-deoxy-2,2-difluoropentofuranos-1-ulose-3-benzoate

To a 2 liter flask fitted with a reflux condenser were added 104 g (0.27 mole) of 96.0% pure ethyl 2,2-difluoro-3-(benzoyloxy)-3-(2,2-dimethyldioxolan-4-yl)propionate which consisted of 3 parts of the 3-R-benzoyloxy enantiomer to 1 part of the 3-S- benzoyloxy enantiomer. Acetonitrile (1000 ml), deionized water (25 ml, 1.35 mole), and trifluoroacetic acid (6.4 g, 0.05 mole) were added. The resulting solution was heated to its reflux temperature (about 78°C), and stirred at that temperature for 4 hours. After 4 hours the condenser was modified in order to allow the boiling liquid to distill rather than reflux. As the volatile acetonitrile, water, and trifluoroacetic acid distilled, fresh dry acetonitrile was added in order to maintain the solution volume at about 1000 ml. After a total of 3000 ml of liquid had distilled the solution was cooled to room temperature (22°C).

The identity of the major components in the solution were characterized by a high performance liquid chromatographic (HPLC) comparison with authentic reference standards. The assay sample was prepared by placing 125 µl of the reaction solution in a 25 ml flask, adding 2 ml of isopropanol, and then diluting the resulting solution to 25 ml with hexane. The column was eluted with an elution solvent comprised of 6% by volume isopropanol and 94% by volume hexane. The column employed was a 25 cm Zorbax CN. The detector had a wavelength of 230 nm, the column flow rate was 2.0 ml/min, the injection volume was 10 µl. The HPLC assay established that the reaction solution contained a product assaying 87.2% by weight of an enantiomeric mixture of D-erythro- and D-threo-2-deoxy-2,2-difluoropentofuranos-1-ulose-3-benzoate. The HPLC assay also indicated the major impurities present were 2.7% by weight unreacted propionate and 5.5% by weight ethyl 2,2-difluoro-3-(benzoyloxy)-4,5-dihydroxypentanoate.

### Example 2

### Preparation of an enantiomeric mixture of D-erythro- and D-threo-2-deoxy-2,2-difluoropentofuranos-1-ulose-3-benzoate

To a 110 liter glass lined reactor were added 64 1 of acetonitrile, 6.00 kg (16.2 mole) of a 3 part 3-R- to 1 part 3-S- enantiomeric mixture of ethyl 2,2-difluoro-3-(benzoyloxy)-3-(2,2-dimethyldioxolan-4-yl)propionate, 0.57 kg (4.4 mole) of trifluoroacetic acid, and 1500 ml (83.3 mole) of purified water. The resulting solution was heated to its reflux temperature (about 78°C), and stirred at that temperature for 5½ hours. Next, 16 1 of a solution of acetonitrile, water, and trifluoroacetic acid were distilled and replaced by 16 1 of toluene. The resulting solution was heated to about 96.5°C and an additional 16 1 of volatiles were distilled. Fresh toluene (16 1) was added and the solution was heated to 96.5°C again. The distillation, addition of fresh toluene, and heat to 96.5°C process was repeated until 107 1 of volatile constituents had distilled. The remaining solution was cooled to room temperature (22°C) while maintaining a slight nitrogen bleed into the reactor to ensure that no moist air could enter the reactor during cooling. The resulting solution, characterized by the HPLC assay described in Example 1, contained a product assaying 81.8% by weight of an enantiomeric mixture of D-erythro- and D-threo-2-deoxy-2,2-difluoropentofuranos-l-ulose-3-benzoate. The HPLC assay indicated the major impurities present were 2.2% by weight unreacted propionate and 7.7% by weight ethyl 2,2-difluoro-3-(benzoyloxy)-4,5-dihydroxypentanoate.

### Example 3

### A. Preparation of an enantiomeric mixture of D-erythro- and D-threo-2-deoxy-2,2-difluoropentofuranos-1-ulose

To a 500 ml flask were added 50.0 g (0.2 mole) of a 3 part 3-R- to 1 part 3-S- enantiomeric mixture of ethyl 2,2-difluoro-3-hydroxy-3-(2,2-dimethyldioxolan-4-yl)propionate, 250 ml of ethanol, and 6.0 ml of 6N sulfuric acid. The resulting solution was heated to its reflux temperature (about 76°C), and stirred at that temperature for 3½ hours. After 3½ hours 100 ml of an ethanol/water mixture were distilled and replaced by 100 ml of fresh ethanol. The solution was cooled to room temperature (22°C) and 4.5 g of anhydrous sodium carbonate were added. Ten minutes later 20 g of 3A molecular sieves were added. The resulting mixture was refrigerated overnight. The next morning the sodium carbonate and the molecular sieves were removed by filtration to provide a solution which, when characterized by the HPLC assay described in Example 1, was found to contain 231 mg of D-erythro- and D-threo-2-deoxy-2,2-difluoropentofuranos-1-ulose per ml of solution. Karl Fisher analysis of the solution indicated the water content was 0.9% by weight water.

## Claims

1. A process for preparing an enantiomeric mixture of erythro and threo lactones of the formula 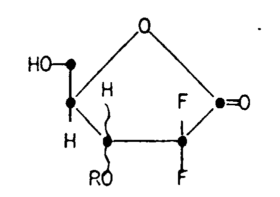 wherein R is H or 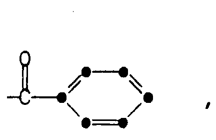 which comprises hydrolyzing a mixture of 3-R- and 3-S- enantiomers of an alkyl 2,2-difluoro-3-hydroxy-3-(2,2-dialkyldioxolan-4-yl)propionate, or a protected derivative thereof, of the formula 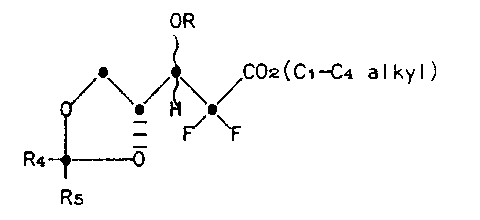 wherein R is as defined above and R⁴ and R⁵ are independently C₁-C₃ alkyl, by using a strong acid as a hydrolytic reagent, followed by azeotropic distillation of water.

## Patentansprüche

1. Verfahren zur Herstellung eines enantiomeren Gemisches aus erythro- und threo-Lactonen der Formel 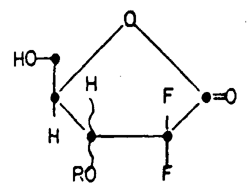 worin R für H oder 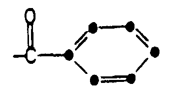 steht, gekennzeichnet durch Hydrolyse eines Gemisches aus 3-R- und 3-S-Enantiomeren eines Alkyl-2,2-difluor-3-hydroxy-3-(2,2-dialkyldioxolan-4-yl)propionats oder eines geschützten Derivats hiervon der Formel 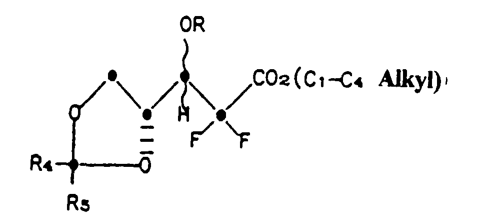 worin R wie oben definiert ist und R⁴ und R⁵ unabhängig für C₁-C₃ Alkyl stehen, durch Verwendung einer starken Säure als Hydrolysereagenz, gefolgt von einer azeotropen Destillation von Wasser.

## Revendications

1. Procédé pour préparer un mélange énantiomère d'érythro- et thréo-lactones répondant à la formule 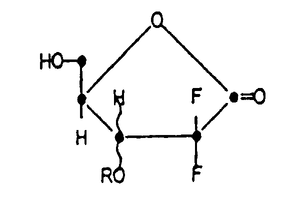 dans laquelle R représente H ou 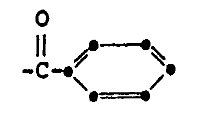 qui comprend le fait d'hydrolyser un mélange d'énantiomères 3-R et 3-S d'un 2,2-difluoro-3-hydroxy-3-(2,2-dialkyldioxolan-4-yl)propionate d'alkyle ou d'un de ses dérivés protégés, répondant à la formule 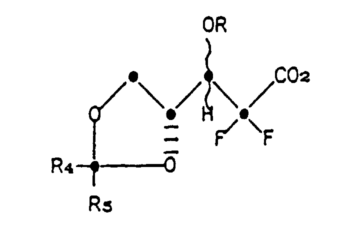 dans laquelle R est tel que défini ci-dessus et R⁴ et R⁵ représentent, indépendamment l'un de l'autre, un groupe alkyle en C₁-C₃, en utilisant un acide fort comme réactif hydrolytique, et en procédant ensuite à une distillation de l'eau par voie azéotrope.
